# EUROPEAN PATENT APPLICATION

(11) **EP 4 231 304 A1**
(43) Date of publication of application: **23.08.2023**
(21) Application number: 21880179.3
(22) Date of filing: 14.10.2021
(51) Int. Cl.: G16C 20/70, G06N 20/00

(54) **INFORMATION PROCESSING DEVICE, INFORMATION PROCESSING METHOD, AND PROGRAM**

(30) Priority: 16.10.2020 JP 2020174415
(71) Applicant: AJINOMOTO CO., INC., Chuo-ku Tokyo 104-8315 (JP)
(72) Inventor: FUJISHIMA, Shohei, Kawasaki-shi, Kanagawa 210-8681 (JP); IKEHIRA, Shu, Kawasaki-shi, Kanagawa 210-8681 (JP)
(74) Representative: Mewburn Ellis LLP
(86) International application number: PCT/JP2021/038010
(87) International publication number: WO 2022/080443

(57) **Abstract**

An object is to provide an information processing apparatus capable of eliminating required trial and error taken by a skilled operator. An information processing apparatus according to an embodiment includes a control unit. The control unit includes a predicting unit that inputs, to a first machine learning model that outputs predicted composition data including a predicted composition value regarding a composition of a thermosetting resin composition when target characteristic data including a characteristic value regarding a characteristic of the thermosetting resin composition is input, or to a second machine learning model that outputs predicted characteristic data including a predicted characteristic value regarding the characteristic of the thermosetting resin composition when target composition data including a target composition value regarding the composition of the thermosetting resin composition is input, the target characteristic data acquired or the target composition data acquired, and acquires the data output from the machine learning model.

## Description

### TECHNICAL FIELD

The present invention relates to an information processing apparatus, an information processing method, and a program.

### BACKGROUND ART

Conventionally, to obtain a desired characteristic or composition in production of a thermosetting resin composition, a preferable characteristic or composition is found by trial and error of a skilled operator.

### PRIOR ART REFERENCES

### PATENT DOCUMENTS

Patent Document 1: JP-A-2020-77346

### DISCLOSURE OF INVENTION

### PROBLEM TO BE SOLVED BY THE INVENTION

However, the characteristic or composition cannot be obtained by the trial and error efficiently. Since a model disclosed in Patent Literature 1 specializes in predicting a composition of a photosensitive resin composition, the model cannot be used in predicting a composition of a thermosetting resin composition that is different in characteristic and composition from a photosensitive resin composition.

The present invention is made in view of the above-mentioned problems, and it is an object of the present invention to provide an information processing apparatus, an information processing method, and a program that can eliminate the need for trial and error by a skilled operator, as well as an information processing apparatus, an information processing method, and a program that can contribute to the elimination of trial and error and produce a machine learning model that predicts a characteristic or composition of a thermosetting resin composition.

### MEANS FOR SOLVING PROBLEM

In order to solve the problem and achieve the object, an information processing apparatus according to one aspect of the present disclosure includes a control unit. The control unit includes a predicting unit that inputs, to a first machine learning model that outputs predicted composition data including a predicted composition value regarding a composition of a thermosetting resin composition when target characteristic data including a characteristic value regarding a characteristic of the thermosetting resin composition is input, or to a second machine learning model that outputs predicted characteristic data including a predicted characteristic value regarding the characteristic of the thermosetting resin composition when target composition data including a target composition value regarding the composition of the thermosetting resin composition is input, the target characteristic data acquired or the target composition data acquired, and acquires the data output from the machine learning model.

An information processing apparatus according to one aspect of the present disclosure includes a control unit. The control unit includes a learning processing unit that uses characteristic data including a characteristic value regarding a characteristic of a thermosetting resin composition and composition data including a composition value regarding a composition of the thermosetting resin composition as learning data to execute a learning process of a first machine learning model that outputs predicted composition data including a predicted composition value regarding the composition of the thermosetting resin composition when target characteristic data including the characteristic value regarding the characteristic of the thermosetting resin composition is input, or a second machine learning model that outputs predicted characteristic data including a predicted characteristic value regarding the characteristic of the thermosetting resin composition when target composition data including a target composition value regarding the composition of the thermosetting resin composition is input.

In the information processing apparatus according to one aspect of the present disclosure, the thermosetting resin composition may have a film shape.

In the information processing apparatus according to one aspect of the present disclosure, the thermosetting resin composition may be for interlayer dielectrics.

In the information processing apparatus according to one aspect of the present disclosure, the composition of the thermosetting resin composition may be presence or absence of at least one of a thermosetting resin, a thermoplastic resin, an inorganic filler, a flame retardant, a curing accelerator, and a solvent, a compound contained in at least one of the thermosetting resin, the thermoplastic resin, the inorganic filler, the flame retardant, the curing accelerator, and the solvent, or a proportion of the compound contained in at least one of the thermosetting resin, the thermoplastic resin, the inorganic filler, the flame retardant, the curing accelerator, and the solvent.

In the information processing apparatus according to one aspect of the present disclosure, the characteristic of the thermosetting resin composition may be at least one of dimensional stability, heat resistance, elasticity, strength, extensibility, dielectric property, surface property, adhesion, electric insulation property, and flame retardance.

An information processing method according to one aspect of the present disclosure is executed by an information processing apparatus including: a control unit. The method executed by the control unit includes: a predicting step of inputting, to a first machine learning model that outputs predicted composition data including a predicted composition value regarding a composition of a thermosetting resin composition when target characteristic data including a characteristic value regarding a characteristic of the thermosetting resin composition is input, or to a second machine learning model that outputs predicted characteristic data including a predicted characteristic value regarding the characteristic of the thermosetting resin composition when target composition data including a target composition value regarding the composition of the thermosetting resin composition is input, the target characteristic data acquired or the target composition data acquired, and acquiring the data output from the machine learning model.

An information processing method according to one aspect of the present disclosure is executed by an information processing apparatus including a control unit. The method includes: a learning processing step of executing a learning process that uses characteristic data including a characteristic value regarding a characteristic of a thermosetting resin composition and composition data including a composition value regarding a composition of the thermosetting resin composition as learning data to execute a first machine learning model that outputs predicted composition data including a predicted composition value regarding the composition of the thermosetting resin composition when target characteristic data including the characteristic value regarding the characteristic of the thermosetting resin composition is input, or a second machine learning model that outputs predicted characteristic data including a predicted characteristic value regarding the characteristic of the thermosetting resin composition when target composition data including a target composition value regarding the composition of the thermosetting resin composition is input. The learning processing step is executed by the control unit.

A program according to one aspect of the present disclosure is for causing an information processing apparatus, including a control unit, to perform, the program. The program includes: a predicting step of inputting, to a first machine learning model that outputs predicted composition data including a predicted composition value regarding a composition of a thermosetting resin composition when target characteristic data including a characteristic value regarding a characteristic of the thermosetting resin composition is input, or a to second machine learning model that outputs predicted characteristic data including a predicted characteristic value regarding the characteristic of the thermosetting resin composition when target composition data including a target composition value regarding the composition of the thermosetting resin composition is input, the target characteristic data acquired or the target composition data acquired, and acquiring the data output from the machine learning model. The predicting step is executed by the control unit.

A program according to one aspect of the present disclosure is for causing an information processing apparatus, including a control unit, to perform, the program. The program includes: a learning processing step of executing a learning process that uses characteristic data including a characteristic value regarding a characteristic of a thermosetting resin composition and composition data including a composition value regarding a composition of the thermosetting resin composition as learning data to execute a first machine learning model that outputs predicted composition data including a predicted composition value regarding a composition of a thermosetting resin composition when target characteristic data including a characteristic value regarding a characteristic of the thermosetting resin composition is input, or a second machine learning model that outputs predicted characteristic data including a predicted characteristic value regarding the characteristic of the thermosetting resin composition when target composition data including a target composition value regarding the composition of the thermosetting resin composition is input. The learning processing step is executed by the control unit.

### EFFECT OF THE INVENTION

The present invention provides an effect of eliminating the need for trial and error by a skilled operator. The present invention provides an effect that can contribute to the elimination of trial and error and produce a machine learning model that predicts a characteristic or composition of a thermosetting resin composition.

### BRIER DESCRIPTION OF DRAWINGS

FIG. 1 is a diagram for explaining an example of a configuration of an information processing apparatus 100;
FIG. 2 is a table for explaining an example of target characteristic data that is input to a first machine learning model and includes a target characteristic value regarding a characteristic of a thermosetting resin composition;
FIG. 3 is a table for explaining an example of predicted composition data that is output from the first machine learning model and includes a predicted composition value regarding the composition of the thermosetting resin composition;
FIG. 4 is a table for explaining an example of composition data for production including a composition value of a thermosetting resin composition for actual production that is designed on the basis of the composition value included in the predicted composition data;
FIG. 5 is a table for explaining an example of measured characteristic data including a characteristic value actually measured for a thermosetting resin composition that is actually produced on the basis of the composition value included in the composition data for production;
FIG. 6 is a table for explaining an example of target composition data that is input to a second machine learning model and includes a target composition value regarding the composition of the thermosetting resin composition;
FIG. 7 is a table for explaining an example of predicted characteristic data that is output from the second machine learning model and includes a predicted characteristic value regarding the characteristic of the thermosetting resin composition;
FIG. 8 is a table for explaining an example of the composition data for production including the composition value of the thermosetting resin composition actually produced that is the same as the composition value included in the target composition data; and
FIG. 9 is a table for explaining an example of the measured characteristic data including the characteristic value actually measured for the thermosetting resin composition actually produced on the basis of the composition value included in the composition data for production.

### BEST MODE(S) FOR CARRYING OUT THE INVENTION

Hereinafter, embodiments of an information processing apparatus, an information processing method, and a program according to the present invention will be explained in detail with reference to the drawings. The present invention is not limited to the embodiments.

### 1. Configuration and Process

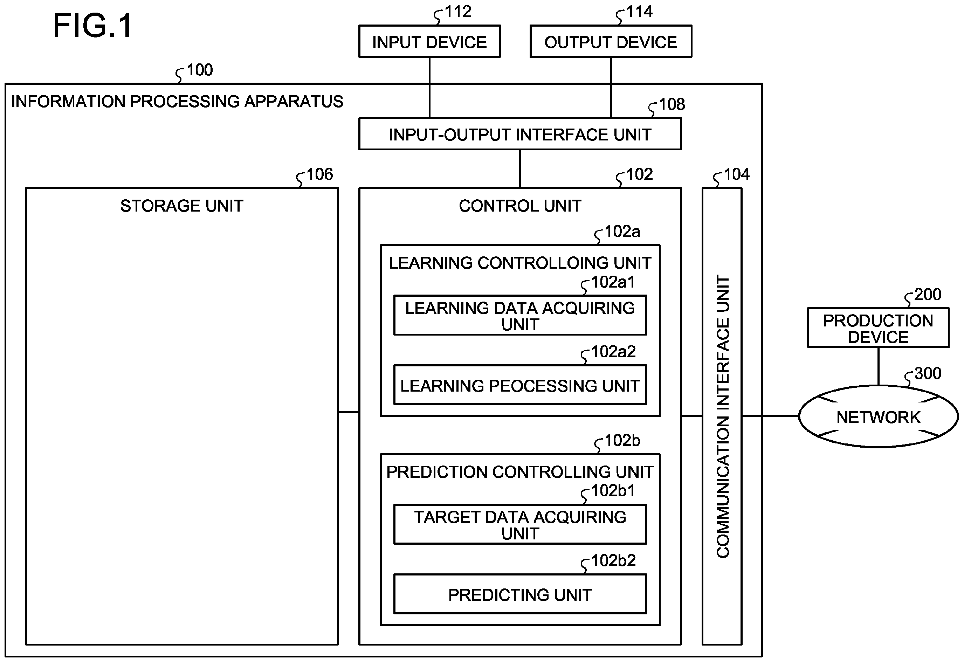

FIG. 1 is a diagram for explaining an example of a configuration of an information processing apparatus 100. The information processing apparatus 100 is communicably connected to a production device 200 that produces a thermosetting resin composition, for example, through a network 300 (e.g., the Internet, intranet, or LAN (local area network, including both wired LAN and wireless LAN)). Herein, the production device 200 may include a mixing unit that mixes a raw material for the thermosetting resin composition into a fluid of the thermosetting resin composition, and a producing unit that produces a thermosetting resin laminated body from the fluid of the thermosetting resin composition.

The information processing apparatus 100 includes a control unit 102 that centrally controls the apparatus, such as a central processing unit (CPU) or a graphics processing unit (GPU), a communication interface unit 104 that communicably connects the apparatus to the network 300 through a communication device such as a router and a wired or wireless communication line such as a dedicated line, a storage unit 106 that stores various databases, tables, files, or the like, and an input-output interface unit 108 that is connected to an input device 112 and an output device 114. The respective units included in the information processing apparatus 100 are communicably connected through any communication path.

The communication interface unit 104 mediates communication between the information processing apparatus 100 and the network 300 (or a communication device such as a router). Specifically, the communication interface unit 104 has a function of communicating data to another terminal through a communication line.

The input-output interface unit 108 is connected to the input device 112 and the output device 114. For the output device 114, a speaker, a printer, or the like can be used in addition to a monitor. For the input device 112, a monitor that implements a pointing device function in cooperation with a mouse, a touch panel, or the like can be used in addition to a keyboard, a mouse, or a microphone.

The storage unit 106 is a storage unit. For the storage unit 106, for example, a memory device such as a random access memory (RAM) or a read only memory (ROM), a fixed disk device such as a hard disk, a flexible disk, or an optical disk can be used. On the storage unit 106, a computer program in which an instruction is given to CPU or GPU in cooperation with an operating system (OS) to execute various processes may be recorded.

The control unit 102 includes an internal memory that stores a control program as an OS or the like, a program defining various processing procedures and the like, required data, and the like, and executes various information processes on the basis of these programs.

The control unit 102 includes a learning controlling unit 102a and a prediction controlling unit 102b based on the concept of function.

The learning controlling unit 102a controls a learning process of a machine learning model. The learning controlling unit 102a includes a learning data acquiring unit 102a1 and a learning processing unit 102a2, and controls the operation of each of the units.

The learning data acquiring unit 102a1 acquires characteristic data and composition data as learning data. The characteristic data includes a characteristic value regarding a characteristic of the thermosetting resin composition. The composition data includes a composition value regarding a composition of the thermosetting resin composition. Herein, the thermosetting resin composition may have, for example, a film shape. The thermosetting resin composition may be, for example, those for interlayer dielectrics. The learning data acquiring unit 102a1 may acquire the characteristic data from an operator, a measurement device (not shown) that measures the characteristic, or an external device (not shown). The learning data acquiring unit 102a1 may acquire the composition data from an operator, the production device 200, or an external device (not shown). Details of the composition data and the characteristic data will be explained in 2. Composition Data and 3. Characteristic Data described below.

The learning processing unit 102a2 uses the characteristic data and the composition data acquired by the learning data acquiring unit 102a1 as the learning data to execute a learning process of a first machine learning model or a second machine learning model. When target characteristic data is input, the first machine learning model outputs predicted composition data. The target characteristic data includes a target characteristic value regarding the characteristic of the thermosetting resin composition. The predicted composition data includes a predicted composition value regarding the composition of the thermosetting resin composition. When target composition data is input, the second machine learning model outputs predicted characteristic data. The target composition data includes a target composition value regarding the composition of the thermosetting resin composition. The predicted characteristic data includes a predicted characteristic value regarding the characteristic of the thermosetting resin composition.

The first machine learning model and the second machine learning model may be a recurrent or time-delay neural network, or the like, or may be based on many machine learning algorithms including a random forest, a gradient boosting, a logistic regression, and a support vector machine (SVM). For example, the first machine learning model may include a node corresponding to each characteristic of the learning data in its input layer, and may include a node corresponding to each raw material of a predicted composition in its output layer. For example, the second machine learning model may include a node corresponding to each composition of the learning data in its input layer, and may include a node corresponding to an indication regarding each predicted characteristic in its output layer. The number of nodes in the input layer relative to one element of the learning data may be one or plural. Between the input layer and the output layer, an intermediate layer (hidden layer) including one or more nodes may be disposed. The learning processing unit 102a2 may adjust the weight of an edge connecting the nodes and a bias of the output node to execute the learning process.

The first machine learning model and the second machine learning model may be stored in a server external to the information processing apparatus 100.

The prediction controlling unit 102b controls a prediction process using a machine learning model. The prediction controlling unit 102b includes a target data acquiring unit 102b1 and a predicting unit 102b2, and controls the operation of each of the units.

The target data acquiring unit 102b1 acquires the target characteristic data or the target composition data. For at least one characteristic, the target characteristic data may include a target numerical range of the characteristic. For at least one composition, the target composition data may include a target numerical range of the composition. The target numerical range may be defined by at least one of an upper limit value and a lower limit value.

The predicting unit 102b2 inputs the target characteristic data acquired by the target data acquiring unit 102b1 to the first machine learning model, and acquires the predicted composition data output from the first machine learning model. The predicting unit 102b2 inputs the target composition data acquired by the target data acquiring unit 102b1 to the second machine learning model, and acquires the predicted characteristic data output from the second machine learning model.

The predicting unit 102b2 may apply bagging (bootstrap aggregating) to the target characteristic data to produce a plurality of pieces of partial target characteristic data in which only a part of the target characteristic is sampled, and input the produced partial target characteristic data to the first machine learning model to acquire a plurality of pieces of predicted composition data. The predicting unit 102b2 may summarize the acquired predicted composition data and acquire a set of the predicted composition data including composition values that may achieve the targets of all the characteristics in the target characteristic data and a target achievement probability of at least a part of the characteristics. The target achievement probability may be a value obtained by multiplying target achievement probabilities of all the characteristics, or a value obtained by weighting the target achievement probabilities of all the characteristics with a weighting factor of each of the characteristics and multiplying the probabilities. The predicting unit 102b2 may use the target characteristic data including the target numerical range of the characteristic to acquire a set of the predicted composition data and a probability distribution of at least one characteristic value.

The predicting unit 102b2 may apply bagging (bootstrap aggregating) to the target composition data to produce a plurality of pieces of partial target composition data in which only a part of the target composition is sampled, and input the produced partial target composition data to the second machine learning model to acquire a plurality of pieces of predicted characteristic data. The predicting unit 102b2 may summarize the acquired predicted characteristic data to acquire a set of the predicted characteristic data including characteristic values that may achieve the targets of all the compositions in the target composition data and a target achievement probability of at least a part of the compositions. The target achievement probability may be a value obtained by multiplying target achievement probabilities of all the compositions, or a value obtained by weighting the target achievement probabilities of all the compositions with a weighting factor of each of the compositions and multiplying the probabilities. The predicting unit 102b2 may use the target composition data including the target numerical range of the composition to acquire a set of the predicted characteristic data and a probability distribution of at least one composition value.

The predicting unit 102b2 may repeatedly use the first machine learning model with a genetic algorithm to acquire the predicted composition data. For example, the predicting unit 102b2 may input the target characteristic data (or the partial target characteristic data) to the first machine learning model to acquire a plurality of pieces of predicted composition data, and then perform any one of selection, crossover, and mutation for the predicted composition data to produce a plurality of pieces of next-generation predicted composition data. The crossover may include as an example exchanging a proportion of at least one part of a raw material between two pieces of predicted composition data. The mutation may include changing the proportion of at least one part of the contained raw material in the predicted composition data. The predicting unit 102b2 may input the produced next-generation predicted composition data to the first machine learning model to acquire a plurality of pieces of characteristic data, and extract, from the predicted composition data input to the first machine learning model, the predicted composition data in which all the characteristics satisfy the target. Hereinafter, the predicting unit 102b2 may perform any one of selection, crossover, and mutation for the extracted predicted composition data to produce a plurality of pieces of next-generation predicted composition data, and repeat a process that extracts, from the predicted composition data, the predicted composition data in which the characteristic data satisfies the target to acquire the predicted composition data in which the target achievement probability exceeds a basic probability.

The predicting unit 102b2 may repeatedly use the second machine learning model with a genetic algorithm to acquire the predicted characteristic data. For example, the predicting unit 102b2 may input the target composition data (or the partial target composition data) to the second machine learning model to acquire a plurality of pieces of predicted characteristic data, and then perform selection for the predicted characteristic data to produce a plurality of pieces of next-generation predicted characteristic data. The predicting unit 102b2 may input the produced next-generation predicted characteristic data to the second machine learning model to acquire a plurality of pieces of composition data, and extract, from the predicted characteristic data input to the second machine learning model, the predicted characteristic data in which all the compositions satisfy the target. Hereinafter, the predicting unit 102b2 may perform selection for the extracted predicted characteristic data to produce a plurality of pieces of next-generation predicted characteristic data, and repeat a process that extracts, from the predicted characteristic data, the predicted characteristic data in which the composition data satisfies the target to acquire the predicted characteristic data in which the target achievement probability exceeds a basic probability.

As described above, the information processing apparatus 100 executes the learning process of the first machine learning model or the second machine learning model using the learning data. When a target characteristic is input to the first machine learning model, a composition for production of a thermosetting resin composition having the target characteristic is output. When a target composition is input to the second machine learning model, a characteristic of a thermosetting resin composition produced with the target composition is output. Therefore, the composition for production of the thermosetting resin composition having the target characteristic can be obtained without requiring trial and error by a skilled operator. Furthermore, the characteristic of the thermosetting resin composition produced with the target composition can be obtained without requiring trial and error by a skilled operator and without measuring.

When the control unit 102 supplies the predicted composition data to the production device 200, a thermosetting resin composition with a predicted composition value included in the predicted composition data may be produced. When the control unit 102 acquires a plurality of pieces of predicted composition data from the predicting unit 102b2, the control unit 102 may display the predicted composition data and supply any one of the predicted composition data selected by an operator to the production device 200. In this case, the control unit 102 may display the acquired predicted composition data in a decreasing order of the target achievement probability. When the operator selects any one characteristic, the control unit 102 may display the predicted composition data in a decreasing order of the target achievement probability of characteristic.

After production of the thermosetting resin composition, the learning controlling unit 102a may relearn the first machine learning model. After measurement of the characteristic of the thermosetting resin composition, the learning controlling unit 102a may relearn the second machine learning model.

### 2. Composition Data

A composition represented by the composition data may be the presence or absence of a raw material that can produce the thermosetting resin composition, or may be a compound contained in the raw material (for example, the name or structural formula of a specific compound contained in the raw material represented by a general name). The composition represented by the composition data may be a proportion of the compound contained in the raw material, or may be a proportion of zero. The raw material that can produce the thermosetting resin composition may contain at least one of a thermosetting resin, a thermoplastic resin, an inorganic filler, a flame retardant, a curing accelerator, and a solvent.

### 2-1. Thermosetting Resin

Examples of the thermosetting resin may include an epoxy resin, a phenol resin, a naphthol resin, a benzoxazine resin, a cyanate ester resin, an active ester resin, a carbodiimide resin, an acid anhydride resin, an oxetane resin, an episulfide resin, an isocyanate resin, an amino resin, a maleimide resin, and a radical curable resin.

Examples of the epoxy resin may include a bisphenol-type epoxy resin, a dicyclopentadiene-type epoxy resin, a trisphenol-type epoxy resin, a naphthol novolac-type epoxy resin, a phenol novolac-type epoxy resin, a tert-butylcatechol-type epoxy resin, a naphthalene-type epoxy resin, a naphthol-type epoxy resin, an anthracenetype epoxy resin, a glycidylamine-type epoxy resin, a glycidyl ester-type epoxy resin, a cresol novolac-type epoxy resin, a biphenyl-type epoxy resin, a linear aliphatic epoxy resin, an epoxy resin having a butadiene structure, an alicyclic epoxy resin, a heterocyclic epoxy resin, a heterocyclic epoxy resin, a spirocycle-containing epoxy resin, a cyclohexane dimethanol-type epoxy resin, a naphthylene ether-type epoxy resin, a trimethylol-type epoxy resin, a tetraphenylethane-type epoxy resin, and a halogenated epoxy resin.

Examples of the phenol resin may include a phenol resin having a novolac structure and a nitrogen-containing phenol resin.

Examples of the naphthol resin may include a naphthol resin having a novolac structure and a nitrogen-containing naphthol resin.

Examples of the benzoxazine resin may include 6,6-(1-methylethylidene)bis(3,4-dihydro-3-phenyl-2H-1,3-benzoxazine), 6,6-(1-methylethylidene)bis(3,4-dihydro-3-methyl-2H-1,3-benzoxazine), and the like.

Examples of the cyanate ester resin may include: a bifunctional cyanate resin such as bisphenol A dicyanate, polyphenolcyanate(oligo(3-methylene-1,5-phenylene cyanate)), 4,4'-methylenebis(2,6-dimethylphenylcyanate), 4,4'-ethylidenediphenyldicyanate, hexafluorobisphenol A dicyanate, 2,2-bis(4-cyanate)phenylpropane, 1,1-bis(4-cyanatephenylmethane), bis(4-cyanate-3,5-dimethylphenyl)methane, 1,3-bis(4-cyanatephenyl-1-(methylethylidene))benzene, bis(4-cyanatephenyl)thioether, and bis(4-cyanatephenyl)ether; a polyfunctional cyanate resin derived from phenol novolac, cresol novolac, and the like; and a prepolymer obtained by partially triazinating these cyanate resins.

Examples of the active ester resin may include a compound having two or more ester groups having high reactivity in one molecule such as phenol esters, thiophenol esters, N-hydroxyamine esters, and esters of heterocyclic hydroxy compounds.

Examples of the carbodiimide resin may include a resin having one or more carbodiimide groups (-N=C=N-) in one molecule.

Examples of the acid anhydride resin may include a resin having one or more acid anhydride groups in one molecule.

Examples of the oxetane resin may include a resin having one or more oxetanyl groups in one molecule.

Examples of the episulphide resin may include a resin having one or more episulphide groups in one molecule.

Examples of the isocyanate resin may include a resin having one or more isocyanate groups in one molecule.

Examples of the amino resin may include a melamine resin, a benzoguanamine resin, an acetoguanamine resin, a spiroguanamine resin, and a cyclohexylguanamine resin.

Examples of the maleimide resin may include an aliphatic maleimide resin and an aromatic maleimide resin.

Examples of the radical curable resin may include a resin that has one or more unsaturated bonds in one molecule and in which a polymerization reaction proceeds due to the presence of a radical species so that a curing reaction proceeds.

### 2-2. Thermoplastic Resin

Examples of the thermoplastic resin may include a phenoxy resin, a polyvinylacetal resin, a polyolefin resin, a polybutadiene resin, a polyimide resin, a polyamideimide resin, a polyetherimide resin, a polysulfone resin, a polyethersulfone resin, a polyphenylene ether resin, a polycarbonate resin, a polyetherketone resin, a polyester resin, and an inden-coumarone resin.

Examples of the phenoxy resin may include a phenoxy resin having one or more skeletons selected from the group consisting of a bisphenol A skeleton, a bisphenol F skeleton, a bisphenol S skeleton, a bisphenol acetophenone skeleton, a novolac skeleton, a biphenyl skeleton, a fluorene skeleton, a dicyclopentadiene skeleton, a norbornene skeleton, a naphthalene skeleton, an anthracene skeleton, an adamantane skeleton, a terpene skeleton, and a trimethylcyclohexane skeleton.

Examples of the polyvinyl acetal resin may include a polyvinyl formal resin, a polyvinyl butyral resin, and a polyvinyl butyral resin.

### 2-3. Inorganic Filler

Examples of the inorganic filler may include silica, alumina, glass, cordierite, silicon oxide, barium sulfate, barium carbonate, talc, clay, mica powder, zinc oxide, hydrotalcite, boehmite, aluminum hydroxide, magnesium hydroxide, calcium carbonate, magnesium carbonate, magnesium oxide, boron nitride, silicon nitride, aluminum nitride, manganese nitride, aluminum borate, strontium carbonate, barium titanate, strontium titanate, calcium titanate, magnesium titanate, bismuth titanate, titanium oxide, zirconium oxide, barium zirconate titanate, barium zirconate, calcium zirconate, zirconium phosphate, and zirconium tungstate phosphate.

Examples of silica may include amorphous silica, ground silica, fused silica, crystalline silica, synthetic silica, hollow silica, and spherical silica.

### 2-4. Flame Retardant

Examples of the flame retardant may include an organophosphorus-based flame retardant, an organic nitrogen-containing phosphorus compound, a nitrogen compound, a silicone-based flame retardant, and a metal hydroxide.

### 2-5. Curing Accelerator

Examples of the curing accelerator may include an imidazole-based curing accelerator, a phosphorus-based curing accelerator, a urea-based curing accelerator, a guanidine-based curing accelerator, a metal-based curing accelerator, an amine-based curing accelerator, and a peroxide-based curing accelerator.

Examples of the imidazole-based curing accelerator may include: an imidazole compound such as 2-methylimidazole, 2-undecylimidazole, 2-heptadecylimidazole, 1,2-dimethylimidazole, 2-ethyl-4-methylimidazole, 2-phenylimidazole, 2-phenyl-4-methylimidazole, 1-benzyl-2-methylimidazole, 1-benzyl-2-phenylimidazole, 1-cyanoethyl-2-methylimidazole, 1-cyanoethyl-2-undecylimidazole, 1-cyanoethyl-2-ethyl-4-methylimidazole, 1-cyanoethyl-2-phenylimidazole, 1-cyanoethyl-2-undecylimidazolium trimellitate, 1-cyanoethyl-2-phenylimidazolium trimellitate, 2,4-diamino-6-[2'-methylimidazolyl-(1')]-ethyl-s-triazine, 2,4-diamino-6-[2'-undecylimidazolyl-(1')]-ethyl-s-triazine, 2,4-diamino-6-[2'-ethyl-4'-methylimidazolyl-(1')]-ethyl-s-triazine, a 2,4-diamino-6-[2'-methylimidazolyl-(1')]-ethyl-s-triazine isocyanuric acid adduct, a 2-phenylimidazole isocyanuric acid adduct, 2-phenyl-4,5-dihydroxymethyl imidazole, 2-phenyl-4-methyl-5-hydroxymethyl imidazole, 2,3-dihydro-1H-pyrrolo[1,2-a]benzimidazole, 1-dodecyl-2-methyl-3-benzylimidazolium chloride, 2-methylimidazoline, and 2-phenylimidazoline; and an adduct of imidazole compounds and epoxy resins.

Examples of the phosphorus-based curing accelerator may include: an aliphatic phosphonium salt such as tetrabutylphosphonium bromide, tetrabutylphosphonium chloride, tetrabutylphosphonium acetate, tetrabutylphosphonium decanoate, tetrabutylphosphonium laurate, bis(tetrabutylphosphonium) pyromellitate, tetrabutylphosphonium hydrogenhexahydrophthalate, tetrabutylphosphonium 2,6-bis[(2-hydroxy-5-methylphenyl)methyl]-4-methylphenolate, and di-tert-butylmethylphosphonium tetraphenylborate; an aromatic phosphonium salt such as methyltriphenylphosphonium bromide, ethyltriphenylphosphonium bromide, propyltriphenylphosphonium bromide, butyltriphenylphosphonium bromide, benzyltriphenylphosphonium chloride, tetraphenylphosphonium bromide, p-tolyl triphenylphosphonium tetra-p-tolyl borate, tetraphenylphosphonium tetraphenyl borate, tetraphenylphosphonium tetra-p-tolyl borate, triphenylethylphosphonium tetraphenylborate, tris(3-methylphenyl)ethylphosphonium tetraphenylborate, tris(2-methoxyphenyl)ethylphosphonium tetraphenylborate, (4-methylphenyl)triphenylphosphonium thiocyanate, tetraphenylphosphonium thiocyanate, and butyltriphenylphosphonium thiocyanate; an aromatic phosphine-borane complex such as triphenylphosphinetriphenylborane; an aromatic phosphine-quinone addition reaction product such as a triphenylphosphine-p-benzoquinone addition reaction product; an aliphatic phosphine such as tributylphosphine, tri-tert-butylphosphine, trioctylphosphine, di-tert-butyl(2-butenyl)phosphine, di-tert-butyl(3-methyl-2-butenyl)phosphine, and tricyclohexylphosphine; an aromatic phosphine such as dibutylphenylphosphine, di-tert-butylphenylphosphine, methyldiphenylphosphine, ethyldiphenylphosphine, butyldiphenylphosphine, diphenylcyclohexylphosphine, triphenylphosphine, tri-o-tolylphosphine, tri-m-tolylphosphine, tri-p-tolylphosphine, tris(4-ethylphenyl)phosphine, tris(4-propylphenyl)phosphine, tris(4-isopropylphenyl)phosphine, tris(4-butylphenyl)phosphine, tris(4-tert-butylphenyl)phosphine, tris(2,4-dimethylphenyl)phosphine, tris(2,5-dimethylphenyl)phosphine, tris(2,6-dimethylphenyl)phosphine, tris(3,5-dimethylphenyl)phosphine, tris(2,4,6-trimethylphenyl)phosphine, tris(2,6-dimethyl-4-ethoxyphenyl)phosphine, tris(2-methoxyphenyl)phosphine, tris(4-methoxyphenyl)phosphine, tris(4-ethoxyphenyl)phosphine, tris(4-tert-butoxyphenyl)phosphine, diphenyl-2-pyridylphosphine, 1,2-bis(diphenylphosphino)ethane, 1,3-bis(diphenylphosphino)propane, 1,4-bis(diphenylphosphino)butane, 1,2-bis(diphenylphosphino)acetylene, and 2,2'-bis(diphenylphosphino)diphenyl ether.

Examples of the urea-based curing accelerator may include: 1,1-dimethylurea; an aliphatic dimethylurea such as 1,1,3-trimethylurea, 3-ethyl-1,1-dimethylurea, 3-cyclohexyl-1,1-dimethylurea, and 3-cyclooctyl-1,1-dimethylurea; and an aromatic dimethylurea such as 3-phenyl-1,1-dimethylurea, 3-(4-chlorophenyl)-1,1-dimethylurea, 3-(3,4-dichlorophenyl)-1,1-dimethylurea, 3-(3-chloro-4-methylphenyl)-1,1-dimeylurea, 3-(2-methylphenyl)-1,1-dimethylurea, 3-(4-methylphenyl)-1,1-dimethylurea, 3-(3,4-dimethylphenyl)-1,1-dimethylurea, 3-(4-isopropylphenyl)-1,1-dimethylurea, 3-(4-methoxyphenyl)-1,1-dimethylurea, 3-(4-nitrophenyl)-1,1-dimethylurea, 3-[4-(4-methoxyphenoxy)phenyl]-1,1-dimethylurea, 3-[4-(4-chlorophenoxy)phenyl]-1,1-dimethylurea, 3-[3-(trifluoromethyl)phenyl]-1,1-dimethylurea, N,N-(1,4-phenylene)bis(N',N'-dimethylurea), and N,N-(4-methyl-1,3-phenylene)bis(N',N'-dimethylurea)[toluene bisdimethylurea].

Examples of the guanidine-based curing accelerator may include dicyandiamide, 1-methylguanidine, 1-ethylguanidine, 1-cyclohexylguanidine, 1-phenylguanidine, 1-(o-tolyl)guanidine, dimethylguanidine, diphenylguanidine, trimethylguanidine, tetramethylguanidine, pentamethylguanidine, 1,5,7-triazabicyclo[4.4.0]deca-5-ene, 7-methyl-1,5,7-triazabicyclo[4.4.0]deca-5-ene, 1-methylbiguanide, 1-ethylbiguanide, 1-n-butylbiguanide, 1-n-octadecylbiguanide, 1,1-dimethylbiguanide, 1,1-diethylbiguanide, 1-cyclohexylbiguanide, 1-allylbiguanide, 1-phenylbiguanide, and 1-(o-tolyl)biguanide.

Examples of the metal-based curing accelerator may include an organometallic complex and an organometallic salt of a metal such as cobalt, copper, zinc, iron, nickel, manganese, and tin.

Examples of the amine-based curing accelerator may include a trialkylamine such as triethylamine and tributylamine, 4-dimethylaminopyridine, benzyldimethylamine, 2,4,6,-tris(dimethylaminomethyl)phenol, and 1,8-diazabicyclo(5,4,0)-undecene.

Examples of the peroxide-based curing accelerator may include t-butyl cumyl peroxide, t-butyl peroxyacetate, α,α'-di(t-butylperoxy)diisopropylbenzene, t-butyl peroxylaurate, t-butyl peroxy-2-ethylhexanoate, t-butyl peroxyneodecanoate, and t-butyl peroxybenzoate.

### 2-6. Solvent

Examples of the solvent may include a ketone-based solvent, an ester-based solvent, an ether-based solvent, an alcohol-based solvent, an ether-ester-based solvent, an ester-alcohol-based solvent, an ether-alcohol-based solvent, an amide-based solvent, a sulfoxide-based solvent, a nitrile-based solvent, an aliphatic hydrocarbon-based solvent, and an aromatic hydrocarbon-based solvent.

Examples of the ketone-based solvent may include acetone, methyl ethyl ketone, methyl isobutyl ketone, and cyclohexanone.

Examples of the ester-based solvent may include methyl acetate, ethyl acetate, butyl acetate, isobutyl acetate, isoamyl acetate, methyl propionate, ethyl propionate, and γ-butyrolactone.

Examples of the ether-based solvent may include tetrahydropyran, tetrahydrofuran, 1,4-dioxane, diethyl ether, diisopropyl ether, dibutyl ether, and diphenyl ether.

Examples of the alcohol-based solvent may include methanol, ethanol, propanol, butanol, and ethylene glycol.

Examples of the ether-ester-based solvent may include 2-ethoxyethyl acetate, propylene glycol monomethyl ether acetate, diethylene glycol monoethyl ether acetate, ethyl diglycol acetate, γ-butyrolactone, and methyl methoxypropionate.

Examples of the ester-alcohol-based solvent may include methyl lactate, ethyl lactate, and methyl 2-hydroxyisobutyrate.

Examples of the ether-alcohol-based solvent may include 2-methoxypropanol, 2-methoxyethanol, 2-ethoxyethanol, propylene glycol monomethyl ether, and diethylene glycol monobutyl ether (butyl carbitol).

Examples of the amide-based solvent may include N,N-dimethylformamide, N,N-dimethylacetamide, and N-methyl-2-pyrrolidone.

Examples of the sulfoxide-based solvent may include dimethyl sulfoxide.

Examples of the nitrile-based solvent may include acetonitrile and propionitrile.

Examples of the aliphatic hydrocarbon-based solvent may include hexane, cyclopentane, cyclohexane, and methylcyclohexane.

Examples of the aromatic hydrocarbon-based solvent may include benzene, toluene, xylene, ethylbenzene, and trimethylbenzene.

### 3. Characteristic Data

A characteristic represented by the characteristic data may be, for example, at least one of dimensional stability, heat resistance, elasticity, strength, extensibility, dielectric property, surface property, adhesion, electric insulation property, and flame retardance of the thermosetting resin composition.

Examples of an indication regarding the dimensional stability of the thermosetting resin composition may include a coefficient of linear thermal expansion CTE a1 (the unit is ppm/K) at a low temperature range (for example, 25 to 150°C), and a coefficient of linear thermal expansion CTE a2 (the unit is ppm/K) at a high temperature range (for example, 150 to 240°C). The CTE a1 may be obtained, for example, by the following method 1. The CTE a2 may be obtained, for example, by the following method 2.

### Method 1

A cured product film for evaluation *1 is cut to obtain a specimen having a width of 5 mm and a length of 15 mm. The obtained specimen is subjected to thermomechanical analysis by a tensile loading method using a thermomechanical analyzer ("Thermo Plus TMA8310" manufactured by Rigaku Corporation). The specimen is mounted on the analyzer, and the coefficient of linear thermal expansion is continuously measured twice under a measurement condition of a load of 1 g and a temperature-increasing rate of 5°C/min. In the second measurement, an average coefficient of linear thermal expansion (ppm/K) at 25°C to 150°C is calculated. Three specimens are subjected to the measurement (n=3), and an average is calculated. *1: The cured product film for evaluation may be produced, for example, by the following procedure 1.

### Procedure 1

A protective film is peeled from a resin sheet *2 and heated at 200°C for 90 minutes in a nitrogen atmosphere to thermally cure a resin composition layer. A support is then peeled to obtain the cured product film for evaluation formed from a cured product of the resin composition. *2: The resin sheet may be produced, for example, by the following procedure 2.

### Procedure 2

A PET film having a main surface release-treated with an alkyd resin-based release agent ("AL-5" available from Lintec Corporation) is prepared as the support. A varnish of the prepared resin composition is uniformly applied to the release-treated surface of the release PET using a die coater so that the thickness of the dried resin composition layer is 40 µm, and dried at 90°C for 3 minutes. Subsequently, a polypropylene film as a protective film is laminated on an exposed surface (a surface not bonded to the release PET) of the resin composition layer. Thus, the resin sheet including the support, the resin composition layer disposed on the support, and the protective film disposed on the resin composition layer is obtained.

### Method 2

A cured product film for evaluation *1 is cut to obtain a specimen having a width of 5 mm and a length of 15 mm. The obtained specimen is subjected to thermomechanical analysis by a tensile loading method using a thermomechanical analyzer ("Thermo Plus TMA8310" manufactured by Rigaku Corporation). The specimen is mounted on the analyzer, and the coefficient of linear thermal expansion is continuously measured twice under a measurement condition of a load of 1 g and a temperature-increasing rate of 5°C/min. In the second measurement, an average coefficient of linear thermal expansion (ppm/K) at 150°C to 240°C is calculated. Three specimens are subjected to the measurement (n=3), and an average is calculated.

Examples of an indication regarding the heat resistance of the thermosetting resin composition include a glass transition temperature Tg (the unit is °C). The Tg may be obtained, for example, by the following method 3 or 4.

### Method 3

A cured product film for evaluation *1 is cut to obtain a specimen having a width of 5 mm and a length of 15 mm. The obtained specimen is subjected to thermomechanical analysis by a tensile loading method using a thermomechanical analyzer ("Thermo Plus TMA8310" manufactured by Rigaku Corporation). The specimen is mounted on the analyzer, and the glass transition temperature is continuously measured twice under a measurement condition of a load of 1 g and a temperature-increasing rate of 5°C/min. In the second measurement, the glass transition temperature Tg (°C) is calculated.

### Method 4

A cured product film for evaluation *1 is cut to obtain a specimen having a width of 5 mm and a length of 15 mm. The viscoelasticity of the obtained specimen is measured by a tensile loading method using a viscoelasticity measurement device ("DMA7100" manufactured by Hitachi High-Tech Corporation). Specifically, the specimen is mounted on the viscoelasticity measurement device, and the storage elastic modulus and the loss elastic modulus are measured under a measurement condition of a load of 200 mN and a temperature-increasing rate of 5°C/min. From a peak value of tanδ (a temperaturedependent curve that is a ratio of the storage elastic modulus and the loss elastic modulus) obtained as a measurement result, the glass transition temperature Tg (°C) is acquired.

Examples of an indication regarding the elasticity of the thermosetting resin composition include an elastic modulus (the unit is GPa). Examples of an indication regarding the strength of the thermosetting resin composition include a strength at a break point (the unit is Mpa). Examples of an indication regarding the extensibility of the thermosetting resin composition include an elongation (the unit is %). The elastic modulus, the strength at a break point, and the elongation may be obtained, for example, by the following method 5.

### Method 5

A cured product film for evaluation *1 is cut to obtain a specimen having a shape of dumbbell No. 1. The tensile strength of the obtained specimen is measured using a tensile testing machine ("RTC-1250A" manufactured by Orientec Co., Ltd.). The elastic modulus, the strength at a break point, and the elongation (elongation at break) at 23°C are measured according to Japanese Industrial Standards (JIS K7127). The measurement is performed five times (n=5), and an average of the highest three values is calculated.

Examples of an indication regarding the dielectric property of the thermosetting resin composition include a specific inductive Dk (no unit) and a dielectric loss tangent Df (no unit). The Dk and the Df may be obtained, for example, by the following method 6.

### Method 6

A cured product film for evaluation *1 is cut to obtain a specimen having a width of 2 mm and a length of 80 mm. The specific inductive Dk and the dielectric loss tangent Df of the obtained specimen are measured at a measurement frequency of 5.8 GHz and a measurement temperature of 23°C by a resonant cavity perturbation method using "HP8362B" manufactured by Agilent Technologies. Two specimens are subjected to the measurement, and an average is calculated.

Examples of an indication regarding the surface property of the thermosetting resin composition include an arithmetic average roughness Ra (the unit is nm). The Ra may be obtained, for example, by the following method 7.

### Method 7

The Ra value of a substrate 1 for evaluation *3 is determined from numerical values obtained at a measurement range of 121 µm × 92 µm using a non-contact type surface roughness meter ("WYKO NT3300" manufactured by Veeco Instruments Inc.) in a VSI contact mode with a 50-times lens. The measurement is performed by determining an average of values at ten points randomly selected. *3: The substrate 1 for evaluation may be produced, for example, by the following procedure 3.

### Procedure 3

The substrate 1 for evaluation is produced by performing the following treatments (1) to (4).
(1) Surface treatment of internal layer circuit substrate: Both surfaces of a copper-clad glass fiber cloth-epoxy resin laminated plate having an internal layer circuit (thickness of copper foil: 18 µm, thickness of substrate: 0.4 mm, "R1515A" manufactured by Panasonic Corporation) are etched to a depth of 1 µm with "CZ8101" available from Mec Co., Ltd. Thus, the copper surfaces are subjected to a roughing treatment.
(2) Laminating resin sheet: The prepared resin sheet *2 is subjected to a lamination treatment on both surfaces of the internal layer circuit board using a batch-type vacuum pressure laminator ("MVLP-500" manufactured by Meiki Co., Ltd.) so that the resin composition layer is bonded to the internal layer circuit board. The lamination treatment is performed by reducing the pressure for 30 seconds to an air pressure of equal to or lower than 13 hPa and then pressure-bonding at 100°C and a pressure of 0.74 MPa for 30 seconds.
(3) Curing of resin composition: After laminating the resin sheet, the resin composition layer is thermally cured under a condition of 100°C for 30 minutes and then 180°C for 30 minutes to form an insulating layer. The support (the release PET) is then peeled to expose the insulating layer.
(4) Roughening treatment: The internal circuit board having the exposed insulating layer is immersed in a swelling liquid ("Swelling Dip Securiganth P" available from Atotech Japan K.K., aqueous sodium hydroxide solution containing diethylene glycol monobutyl ether) at 60°C for 10 minutes, then immersed in an oxidant ("Concentrate Compact CP" available from Atotech Japan K.K., aqueous solution of about 6% by mass potassium permanganate and about 4% by mass sodium hydroxide) at 80°C for 20 minutes, and finally immersed in a neutralization liquid ("Reduction Solution Securiganth P" available from Atotech Japan K.K., aqueous hydroxylamine sulfate solution) at 40°C for 5 minutes. The internal circuit board is then dried at 80°C for 30 minutes. Thus, the substrate 1 for evaluation is produced.

Examples of an indication regarding the adhesion of the thermosetting resin composition include plating adhesion strength (the unit is kgf/cm) and copper foil adhesion strength (the unit is kgf/cm). The plating adhesion strength may be obtained, for example, by the following method 8. The copper foil adhesion strength may be obtained, for example, by the following method 9.

### Method 8

A portion having a width of 10 mm and a length of 100 mm of a conductor layer of a substrate 2 for evaluation *4 is cut, and one end of this portion is peeled and gripped by a gripper ("AC-50C-SL" manufactured by TSE Co., Ltd.). The load (kgf/cm) when the end is drawn and peeled using a tensile tester ("AC-50C-SL" manufactured by TSE Co., Ltd.) to a length of 35 mm in a vertical direction at a rate of 50 mm/min is measured at room temperature according to Japanese Industrial Standards (JIS C6481). The peel strength is determined.
*4: The substrate 2 for evaluation may be produced, for example, by the following procedure 4.

### Procedure 4

The substrate 2 for evaluation is produced by performing the treatments (1) to (4) described above and the following treatment (5).

(5) Formation of plating conductive layer: The substrate 1 for evaluation is immersed in a PdCl₂-containing electroless-plating liquid at 40°C for 5 minutes, and then immersed in an electroless copper plating liquid at 25°C for 20 minutes. Subsequently, the substrate 1 is heated at 150°C for 30 minutes to perform an annealing treatment. An etching resist is then formed, and a pattern is formed by etching. After that, copper sulfate electrolytic plating is performed to form a conductive layer having a thickness of 30 µm, and an annealing treatment is performed at 200°C for 60 minutes. Thus, the substrate 2 for evaluation is produced.

### Method 9

A sample for evaluation of copper foil adhesion strength *5 is cut into a piece of 150 × 30 mm. A portion having a width of 10 mm and a length of 100 mm of a copper foil portion of the piece is cut using a cutter, and one end in the longitudinal direction of the copper foil is peeled and gripped by a gripper ("AC-50C-SL" manufactured by TSE Co., Ltd.). The load (kgf/cm) when the end is drawn and peeled to a length of 35 mm in a vertical direction at a rate of 50 mm/min is measured at room temperature using an INSTRON universal testing machine according to Japanese Industrial Standards (JIS C6481).
*5: The sample for evaluation of copper foil adhesion strength may be produced, for example, by the following procedure 5.

### Procedure 5

A sample for evaluation of copper foil adhesion strength is produced by performing the following treatments (1) to (2).
(1) Surface treatment of copper foil: A gloss surface of an electrolytic copper foil ("3EC-III" available from MITSUI MINING & SMELTING CO., LTD., thickness: 35 µm) is immersed in a microetching agent ("CZ-8101" available from Mec Co., Ltd.) to perform a roughening treatment (Ra = 1 µm) of the copper surface. Subsequently, the copper foil is subjected to an anticorrosive treatment using an anticorrosive solution ("CL8300" available from Mec Co., Ltd.). The obtained copper foil is called CZ copper foil. Furthermore, the copper foil is heated in an oven at 130°C for 30 minutes.
(2) Production of sample for evaluation: A copper-clad glass fiber cloth-epoxy resin laminated plate having an internal layer circuit (thickness of copper foil: 18 µm, thickness of substrate: 0.4 mm, "R1515A" manufactured by Panasonic Corporation) is prepared as an internal layer circuit board. Subsequently, the prepared resin sheet *2 is laminated on both surfaces of the internal layer circuit board using a batch-type vacuum pressure laminator ("MVLP-500" manufactured by Meiki Co., Ltd.) so that the resin composition layer is bonded to the internal layer circuit board. The laminating is performed by reducing the pressure for 30 seconds to an air pressure of equal to or lower than 13 hPa and then pressure-bonding at 100°C and a pressure of 0.74 MPa for 30 seconds. After the laminating, the support (the release PET) is peeled. On the exposed resin composition layer, a treated surface of the CZ copper foil is laminated under the same condition as described above. Subsequently, the resin composition layer is cured under a curing condition of 190°C and 90 minutes to form an insulating layer. Thus, the sample for evaluation of copper foil adhesion strength having a structure of CZ copper foil/insulating layer/copper-clad glass fiber cloth-epoxy resin laminated plate/insulating layer/CZ copper foil is produced.

Examples of an indication regarding the electric insulation property of the thermosetting resin composition include a value showing whether insulating can be maintained for 300 hours in an inter-wire b-HAST test (for example, a value showing that insulating can be maintained or a value showing that insulating cannot be maintained).

Examples of an indication regarding the flame retardant of the thermosetting resin composition include a value showing whether V0 can be achieved when a film having a predetermined thickness is laminated on both surfaces of an FR4 substrate and cured (for example, a value showing that V0 can be achieved or a value showing that V0 cannot be achieved).

### 4. Other Embodiments

The embodiments of the present invention are explanted thus far. However, the present invention may be implemented in various different embodiments other than the aforementioned embodiments within the scope of the technical idea described in claims.

For example, of the processes explained in the embodiments, all or a part of the processes explained as a process that is automatically performed can also be performed manually, or all or a part of the processes explained as a process that is manually performed can also be performed automatically by a publicly-known method.

In addition, the processing procedures, controlling procedures, specific names, information including registration data of the respective processes and parameters in search conditions, screen examples, and database configuration shown in the aforementioned documents and the drawings can be optionally changed unless otherwise specified.

Components shown in the drawings of the devices are function conceptual components and do not necessarily need to be physically configured as shown in the drawings.

For example, all or a part of a processing function of the information processing apparatus 100, particularly each processing function performed by the control unit 102, may be achieved by a CPU or a GPU and a program interpreted and executed by the CPU or the GPU, or may be achieved as a hardware by wired logic. The program is recorded on a non-transitory computer readable recording medium including a programmed instruction that allows the information processing apparatus to perform the information processing method according to the present invention, and is mechanically read by the information processing apparatus 100, if necessary. In other words, a computer program that gives an instruction to the CPU or the GPU in cooperation with an OS and performs various processes is recorded on the storage unit 106 such as a ROM or a hard disk drive (HDD). This computer program is executed by loading the program into the RAM, and constitutes the control unit in cooperation with the CPU or the GPU.

This computer program may be stored in an application program server connected to the information processing apparatus 100 through any network, and all or a part of the computer program can be downloaded, if necessary.

The program according to the present invention may be stored in a non-transitory computer readable recording medium, or may be constituted as a program product. This "recording medium" includes an optional "portable physical medium" such as a memory card, a USB memory, an SD card, a flexible disk, a magneto-optical disk, a ROM, an erasable programmable read only memory (EPROM), an electrically erasable and programmable read only memory (EEPROM), a compact disk read only memory (CD-ROM), an MO, a digital versatile disk (DVD), and a Blu-ray (registered trademark) disc.

The "program" is a data processing method described in optional language or description method, and any form such as a source code or a binary code is acceptable. The "program" is not necessarily limited to a program configured as a single unit, and includes those distributedly configured as a plurality of modules and libraries and those in which the function of the program is achieved in cooperation with separate programs typified by an OS. For a specific configuration and a reading procedure to read a recording medium by the devices shown in the embodiments, and an installation procedure after the reading, or the like, known configuration and procedures can be used.

Various databases and the like stored in the storage unit 106 are a storage unit including a memory device such as RAM and ROM, a fixed disk such as a hard disk, a flexible disk, and an optical disk, and store various programs, tables, databases, files for web pages, and the like that are used for various processes and providing websites.

The information processing apparatus 100 may be configured as an information processing apparatus such as a known personal computer or a work station, or may be configured as the information processing apparatus connected to an optional peripheral device. The information processing apparatus 100 may be achieved by installing software (including the program or the data) that allows the information processing apparatus to implement the information processing method according to the present invention.

A specific configuration of distribution or integration of the devices is not limited to those shown in the drawings. The devices can be configured by functionally or physically distributing or integrating all or a part of the devices in optional units according to various additions or the like or according to functional loads. In other words, the aforementioned embodiments may optionally be combined for implementation, or the embodiments may be selectively implemented.

### Examples

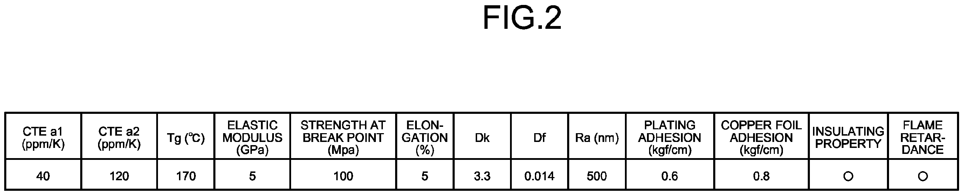

An example in which predicted composition data was acquired from target characteristic data using a first machine learning model after a pre-learning treatment will be explained with reference to FIGs. 2 to 5.

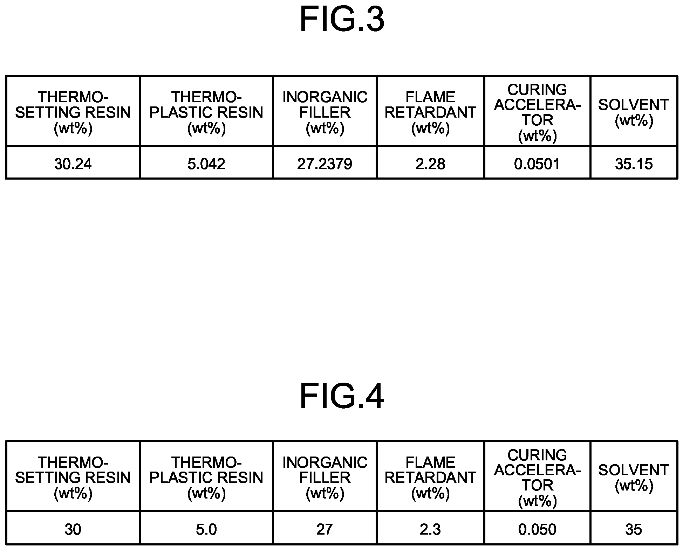

To the first machine learning model after a pre-learning treatment, the target characteristic data shown in FIG. 2 was input, and as a result, the predicted composition data shown in FIG. 3 was output from the first machine learning model. In FIG. 2, a symbol "O" described in a column of insulating property represents that insulating can be maintained for 300 hours in the inter-wire b-HAST test, and a symbol "O" described in a column of flame retardance represents that V0 can be achieved when a film having a predetermined thickness is laminated on both surfaces of an FR4 substrate and cured. For a method for measuring each characteristic, 3. Characteristic Data shall be referenced.

Based on the output predicted composition data shown in FIG. 3, the composition data for production including a composition value of each composition of a thermosetting resin composition to be actually produced as shown in FIG. 4 was prepared within an allowable range of the composition value ± 1%.

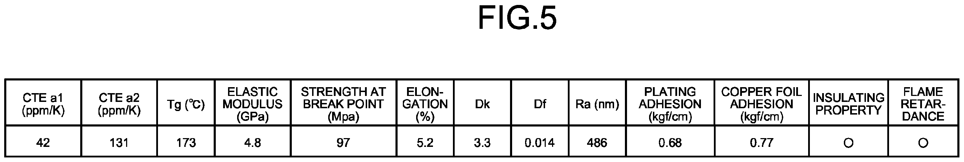

Based on the prepared composition data for production shown in FIG. 4, the thermosetting resin composition was actually produced. The characteristic of the produced thermosetting resin composition was measured, and as a result, measured characteristic data shown in FIG. 5 was obtained. In FIG. 5, a symbol "O" described in a column of insulating property represents that insulating can be maintained for 300 hours in the inter-wire b-HAST test, and a symbol "O" described in a column of flame retardance represents that V0 can be achieved when a film having a predetermined thickness is laminated on both surfaces of an FR4 substrate and cured. For a method for measuring each characteristic, 3. Characteristic Data shall be referenced.

The target characteristic data shown in FIG. 2 was compared with the measured characteristic data shown in FIG. 5, and as a result, the measured characteristic data shown in FIG. 5 was confirmed to be very similar to the target characteristic data shown in FIG. 2.

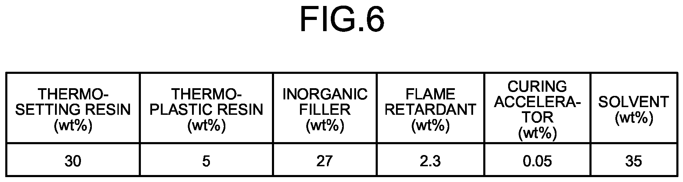

An example in which predicted characteristic data was acquired from target composition data using a second machine learning model after pre-learning will be explained with reference to FIGs. 6 to 9.

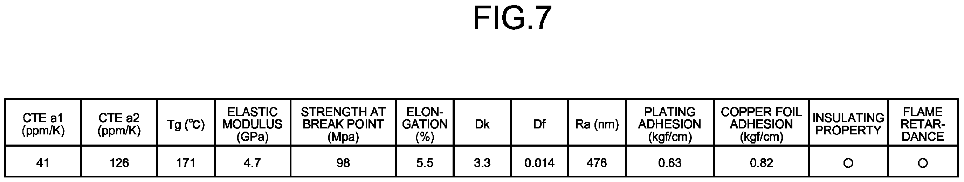

To the second machine learning model after pre-learning, the target composition data shown in FIG. 6 was input, and as a result, the predicted characteristic data shown in FIG. 7 was output from the second machine learning model. In FIG. 7, a symbol "O" described in a column of insulating property represents that insulating can be maintained for 300 hours in the inter-wire b-HAST test, and a symbol "O" described in a column of flame retardance represents that V0 can be achieved when a film having a predetermined thickness is laminated on both surfaces of an FR4 substrate and cured. For a method for measuring each characteristic, 3. Characteristic Data shall be referenced.

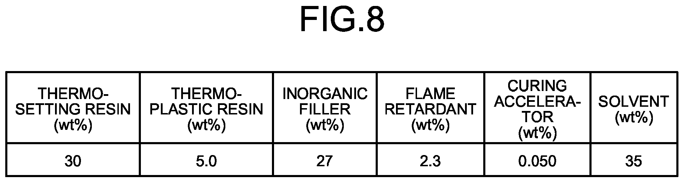

Composition data for production including a composition value of a thermosetting resin composition to be actually produced as shown in FIG. 8 that was the same as the composition value included in the target composition data was prepared.

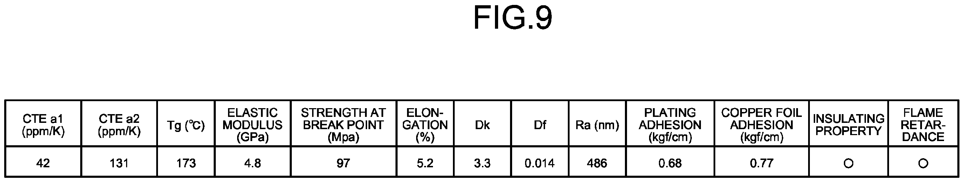

Based on the prepared composition data for production shown in FIG. 8, the thermosetting resin composition was actually produced. The characteristic of the produced thermosetting resin composition was measured, and as a result, measured characteristic data shown in FIG. 9 was obtained. In FIG. 9, a symbol "O" described in a column of insulating property represents that insulating can be maintained for 300 hours in the inter-wire b-HAST test, and a symbol "O" described in a column of flame retardance represents that V0 can be achieved when a film having a predetermined thickness is laminated on both surfaces of an FR4 substrate and cured. For a method for measuring each characteristic, 3. Characteristic Data shall be referenced.

The predicted characteristic data shown in FIG. 7 was compared with the measured characteristic data shown in FIG. 9, and as a result, the measured characteristic data shown in FIG. 9 was confirmed to be very similar to the predicted characteristic data shown in FIG. 7.

### INDUSTRIAL APPLICABILITY

The present invention is extremely useful in producing a thermosetting resin composition or in measuring a characteristic of a thermosetting resin composition.

### EXPLANATIONS OF LETTERS OR NUMERALS

- 100: information processing apparatus
- 102: control unit
- 102a: learning controlling unit
- 102a1: learning data acquiring unit
- 102a2: learning processing unit
- 102b: prediction controlling unit
- 102b1: target data acquiring unit
- 102b2: predicting unit
- 104: communication interface unit
- 106: storage unit
- 108: input-output interface unit
- 112: input device
- 114: output device
- 200: production device
- 300: network

## Claims

1. An information processing apparatus comprising:
a control unit, wherein
the control unit includes a predicting unit that
inputs, to a first machine learning model that outputs predicted composition data including a predicted composition value regarding a composition of a thermosetting resin composition when target characteristic data including a characteristic value regarding a characteristic of the thermosetting resin composition is input or to a second machine learning model that outputs predicted characteristic data including a predicted characteristic value regarding the characteristic of the thermosetting resin composition when target composition data including a target composition value regarding the composition of the thermosetting resin composition is input, the target characteristic data acquired or the target composition data acquired, and
acquires the data output from the machine learning model.

2. An information processing apparatus comprising:
a control unit, wherein
the control unit includes a learning processing unit that uses characteristic data including a characteristic value regarding a characteristic of a thermosetting resin composition and composition data including a composition value regarding a composition of the thermosetting resin composition as learning data to execute a learning process of
a first machine learning model that outputs predicted composition data including a predicted composition value regarding the composition of the thermosetting resin composition when target characteristic data including the characteristic value regarding the characteristic of the thermosetting resin composition is input, or
a second machine learning model that outputs predicted characteristic data including a predicted characteristic value regarding the characteristic of the thermosetting resin composition when target composition data including a target composition value regarding the composition of the thermosetting resin composition is input.

3. The information processing apparatus according to claim 1 or 2, wherein
the thermosetting resin composition has a film shape.

4. The information processing apparatus according to any one of claims 1 to 3, wherein
the thermosetting resin composition is for interlayer dielectrics.

5. The information processing apparatus according to any one of claims 1 to 4, wherein
the composition of the thermosetting resin composition is
presence or absence of at least one of a thermosetting resin, a thermoplastic resin, an inorganic filler, a flame retardant, a curing accelerator, and a solvent,
a compound contained in at least one of the thermosetting resin, the thermoplastic resin, the inorganic filler, the flame retardant, the curing accelerator, and the solvent, or
a proportion of the compound contained in at least one of the thermosetting resin, the thermoplastic resin, the inorganic filler, the flame retardant, the curing accelerator, and the solvent.

6. The information processing apparatus according to any one of claims 1 to 5, wherein
the characteristic of the thermosetting resin composition is at least one of dimensional stability, heat resistance, elasticity, strength, extensibility, dielectric property, surface property, adhesion, electric insulation property, and flame retardance.

7. An information processing method executed by an information processing apparatus including a control unit, the method executed by the control unit comprising:
a predicting step of
inputting, to a first machine learning model that outputs predicted composition data including a predicted composition value regarding a composition of a thermosetting resin composition when target characteristic data including a characteristic value regarding a characteristic of the thermosetting resin composition is input or to a second machine learning model that outputs predicted characteristic data including a predicted characteristic value regarding the characteristic of the thermosetting resin composition when target composition data including a target composition value regarding the composition of the thermosetting resin composition is input, the target characteristic data acquired or the target composition data acquired, and
acquiring the data output from the machine learning model.

8. An information processing method executed by an information processing apparatus including a control unit, the method executed by the control unit comprising:
a learning processing step of executing a learning process that uses characteristic data including a characteristic value regarding a characteristic of a thermosetting resin composition and composition data including a composition value regarding a composition of the thermosetting resin composition as learning data to execute:
a first machine learning model that outputs predicted composition data including a predicted composition value regarding the composition of the thermosetting resin composition when target characteristic data including the characteristic value regarding the characteristic of the thermosetting resin composition is input, or
a second machine learning model that outputs predicted characteristic data including a predicted characteristic value regarding the characteristic of the thermosetting resin composition when target composition data including a target composition value regarding the composition of the thermosetting resin composition is input.

9. A program for causing an information processing apparatus including a control unit to perform a method, the method executed by the control unit comprising:
a predicting step of
inputting, to a first machine learning model that outputs predicted composition data including a predicted composition value regarding a composition of a thermosetting resin composition when target characteristic data including a characteristic value regarding a characteristic of the thermosetting resin composition is input or a to second machine learning model that outputs predicted characteristic data including a predicted characteristic value regarding the characteristic of the thermosetting resin composition when target composition data including a target composition value regarding the composition of the thermosetting resin composition is input, the target characteristic data acquired or the target composition data acquired, and
acquiring the data output from the machine learning model.

10. A program for causing an information processing apparatus including a control unit to perform a method, the method executed by the control unit comprising:
a learning processing step of executing a learning process that uses characteristic data including a characteristic value regarding a characteristic of a thermosetting resin composition and composition data including a composition value regarding a composition of the thermosetting resin composition as learning data to execute:
a first machine learning model that outputs predicted composition data including a predicted composition value regarding a composition of a thermosetting resin composition when target characteristic data including a characteristic value regarding a characteristic of the thermosetting resin composition is input, or
a second machine learning model that outputs predicted characteristic data including a predicted characteristic value regarding the characteristic of the thermosetting resin composition when target composition data including a target composition value regarding the composition of the thermosetting resin composition is input.
